# EUROPEAN PATENT SPECIFICATION

(11) **EP 3 779 452 B1**
(45) Date of publication and mention of the grant of the patent: **03.05.2023**
(21) Application number: 19776653.8
(22) Date of filing: 29.03.2019
(51) Int. Cl.: G01N 33/68

(54) **NEONATAL HYPOXIC-ISCHEMIC ENCEPHALOPATHY SEVERITY DETERMINING METHOD AND PROGNOSIS PREDICTING METHOD**
VERFAHREN UND METHODE ZUR BESTIMMUNG DES SCHWEREGRADES VON NEONATALER HYPOXISCH-ISCHÄMISCHER ENZEPHALOPATHIE
PROCÉDÉ JUGEANT LA GRAVITÉ AINSI QUE PROCÉDÉ PRÉVOYANT LES CONSÉQUENCES D'UNE ENCÉPHALOPATHIE HYPOXIQUE ISCHÉMIQUE DU NOURRISSON

(30) Priority: 30.03.2018 JP 2018070037
(43) Date of publication of application: 17.02.2021
(73) Proprietor: National Center of Neurology and Psychiatry, Kodaira-shi Tokyo 187-8551 (JP)
(72) Inventor: ITO, Masayuki, Tokyo 187-8551 (JP); AKAMATSU, Tomohisa, Tokyo 187-8551 (JP)
(74) Representative: J A Kemp LLP
(86) International application number: PCT/JP2019/013927
(87) International publication number: WO 2019/189725

(56) References cited:
- EP-A1- 2 896 702
- WO-A1-2015/125752
- JP-A- 2013 545 087
- JP-A- 2015 147 753
- KAZUTAKA HAYASHIDA ET AL: "Serum Soluble Lectin-Like Oxidize Low-Density Lipoprotein Receptor-1 levels Are Elevated in Acute Coronary Syndrome A Novel Marker foe early Diagnosis", CIRCULATION, AMERICAN HEART ASSOCIATION, US, vol. 112, no. 6, 9 August 2005 (2005-08-09), pages 812-818, XP003014705, ISSN: 0009-7322, DOI: 10.1161/CIRCULATIONAHA.104.468397
- INOUE NOBUTAKA ET AL: "LOX Index, a Novel Predictive Biochemical Marker for Coronary Heart Disease and Stroke", CLINICAL CHEMISTRY, vol. 56, no. 4, 1 April 2010 (2010-04-01), pages 550-558, XP055862035, US ISSN: 0009-9147, DOI: 10.1373/clinchem.2009.140707 Retrieved from the Internet: URL:https://watermark.silverchair.com/clin chem0550.pdf?token=AQECAHi208BE49Ooan9kkhW _Ercy7Dm3ZL_9Cf3qfKAc485ysgAAAu8wggLrBgkqh kiG9w0BBwagggLcMIIC2AIBADCCAtEGCSqGSIb3DQE HATAeBglghkgBZQMEAS4wEQQMqolIppve94rpwsGWA gEQgIICoovgCarEBKF9zpeipn1Wv4hXeopXOIOqlbn E6e46XBY7x91Cst-fDAgf2rcAW10YaYvOisTd0M-_4 irRXyg9W2u>
- AKAMATSU, T.: "LOX-1 is a novel therapeutic target in neonatal hypoxic-ischemic encephalopathy", THE AMERICAN JOURNAL OF PATHOLOGY, vol. 184, no. 6, June 2014 (2014-06), pages 1843-1852, XP055638837,
- YOKOTA, C. et al.: "High Levels of Soluble Lectin- Like Oxidized Low-Density Lipoprotein Receptor-1 in Acute Stroke: An Age- and Sex-Matched Cross- Sectional Study", J ATHEROSCLER THROMB., vol. 23, no. 10, 2016, pages 1222-1226, XP055638847,
- CHAO, L. et al.: "Correlation between pulse wave velocity and serum sLOX-1 in patients with acute cerebral infarction", Sci Res Essays ., vol. 6, no. 31, 16 December 2011 (2011-12-16), pages 6515-6519, XP055749607,
- AKAMATSU, T.: "A Pilot Study of Soluble Form of LOX-1 as a Novel Biomarker for Neonatal Hypoxic-Ischemic Encephalopathy", THE JOURNAL OF PEDIATRICS, vol. 206, 12 December 2018 (2018-12-12), pages 49-55, XP085610897,
- Tomohisa Akamatsu: "O-045. sLOX-1 as a Novel Biomarker of Neonatal Hypoxic-Ischemic Encephalopathy", Journal of Japan Society of Perinatal and Neonatal Medicine, vol. 54, no. 2, 30 June 2018 (2018-06-30), page 502, XP009524166, ISSN: 1348-964X

## Description

### Technical Field

The present invention relates to a method for determining the severity of neonatal hypoxic-ischemic encephalopathy in a newborn affected with the disease as a subject, and a method for predicting the prognosis after treatment by therapeutic hypothermia, in a newborn affected with the disease.

### Background Art

Neonatal hypoxic-ischemic encephalopathy (often referred to as "nHIE" in this specification) is cerebrovascular disorder that develops unexpectedly in newborns due to severe neonatal asphyxia, acute respiratory-circulatory failure and fetal hypoxia or the like. The nHIE is a disease with generally poor prognosis, and although incidence ranges from 0.1% to 0.6%, the fatality rate ranges from 10% to 60%, and the incidence of neurological sequelae such as cerebral palsy and epilepsy is extremely high as 25% (Non-patent Literatures 1 to 3). Neurotoxicity of the excitatory neurotransmitter glutamate released into the neuronal gaps due to hypoxia and ischemia, and neuronal injuries and cerebral edema due to inflammatory substances such as cytokines are deeply related to the pathological conditions (Non-patent Literatures 4 and 5). Therefore, for the therapy for the nHIE, treatment during the latent phase before irreversible cell damage occurs, that is, usually, treatment within 6 hours after birth is important.

At present, only the therapeutic hypothermia has been proven to have a neuroprotective effect on nHIE (Non-Patent Literatures 6 to 11). However, therapeutic hypothermia requires special equipment such as a cooling device, a ventilator, and an electroencephalogram monitor in which the procedures and management are very complicated, and thus can be performed only at specific advanced medical institutions. However, neonatal asphyxia and nHIE can develop unexpectedly in every birthing facility, and the newborn has to be sent to an advanced medical institution for performing therapeutic hypothermia. On the other hand, nHIE has progressed since before or at the time of birth, and even therapeutic hypothermia is not effective unless the treatment is started within 6 hours after birth, as described above. That is, in order to obtain an effective therapeutic effect by therapeutic hypothermia, a newborn must be diagnosed whether or not the newborn is affected with nHIE within 6 hours after birth. However, no nHIE detection method, which can be performed early and quickly after the birth of a newborn, has been developed so far.

In order to solve the problem, the present inventors have prepared a nHIE model rat, and have identified 12 types of genes and the gene products thereof, wherein the expression thereof is significantly increased or decreased upon the onset of nHIE, and conversely such increase or decrease is cancelled by performing therapeutic hypothermia, and thus have developed them as biomarkers for detecting cerebrovascular disorders (Patent Literature 1).

### Citation List

### Patent Literature

Patent Literature 1: WO2015/125752

### Non Patent Literature

Non Patent Literature 1: Shankaran S, 2009, J Neurotrauma. 26(3):437-443. doi: 10.1089/ neu.2008.0678.
Non Patent Literature 2: Gunn AJ., 2000, Curr Opin Pediatr. 12(2):111-115.
Non Patent Literature 3: Vannucci RC & Perlman JM, 1997, Pediatrics. 100(6):1004-1014.
Non Patent Literature 4: Drury PP, et al., 2010, Semin Fetal Neonatal Med. 15(5):287-292. doi: 10.1016/j.siny.2010.05.005.
Non Patent Literature 5: Johnston MV, et al., 2011, Lancet Neurol. 10(4):372-82. doi: 10.1016/S 1474-4422(11)70016-3.
Non Patent Literature 6: Gluckman PD, et al., 2005, Lancet. 365(9460):663-670.
Non Patent Literature 7: Shankaran S, et al., 2005, N Engl J Med. 353(15):1574-1584.
Non Patent Literature 8: Azzopardi DV, et al., 2009, N Engl J Med. 361(14):1349-1358. doi: 10.1056/NEJMoa0900854.
Non Patent Literature 9: Simbruner G, et al., 2010, Pediatrics. 126(4): e771-778. doi: 10.1542/peds.2009-2441.
Non Patent Literature 10: Zhou WH, et al., 2010, J Pediatr. 157(3):367-372, 372.e1-3. doi: 10.1016/j.jpeds.2010.03.030.
Non Patent Literature 11: Jacobs SE, et al., 2011, Arch Pediatr Adolesc Med. 165(8):692-700. doi: 10.1001/archpediatrics.2011.43.

### Summary of Invention

### Technical Problem

The invasiveness of therapeutic hypothermia for newborns is never low. Therefore, this therapy should be applied to an nHIE-affected newborn who need it. However, if the severity is mild, the therapy is not applicable even for an nHIE-affected newborn. Further, in a severe nHIE-affected newborn, improvement is limited and the prognosis is often poor, even if therapeutic hypothermia is applied, and it remains unpredictable even after the application. Therefore, when a newborn as a subject is affected with nHIE, the severity is extremely important information for predicting the necessity of the application of therapeutic hypothermia and the prognosis after applying the therapy.

Using the cerebrovascular disorder detection marker disclosed in Patent Literature 1, it is possible to determine the presence or the absence of nHIE in a newborn. However, the invention disclosed in Patent Literature 1 could not predict the severity of nHIE and the prognostic state after treatment by therapeutic hypothermia.

### Solution to Problem

The present inventors have classified 72 newborns admitted to the neonatal intensive care unit (NICU) into 4 groups: a normal group, a nHIE mild group, a nHIE moderate group, and a nHIE severe group according to the nHIE diagnostic criteria of the US NICHD (National Institute of Child Health and Human Development) and Sarnat Classification for the severity of nHIE (Sarnat Grading scale). As a result of measuring the amount of a soluble peptide in a LOX-1 protein comprised in the plasma collected from newborns of each group within 6 hours after birth, it has been found to be closely related to the severity of nHIE and the prognosis after treatment by therapeutic hypothermia. It has been revealed that the severity of nHIE in newborns and the prognosis after treatment by therapeutic hypothermia can be predicted with high specificity by specifying the amount of a soluble LOX-1 (abbreviated as sLOX-1) protein per unit volume of plasma as a cut-off value. The present invention has been developed based on these new findings, and is defined in the appended claims.

The present specification comprises the contents disclosed in Japanese Patent Application No. 2018-070037, on which the priority of the present application is based. Advantageous Effects of Invention

According to the method for determining the severity of nHIE of the present invention, the severity of nHIE in a nHIE-affected newborn, who is a subject, can be determined.

According to the method for determining the necessity of the application of therapeutic hypothermia of the present invention, whether or not therapeutic hypothermia should be applied to a nHIE-affected newborn, who is a subject, can be determined.

According to the method for predicting the prognosis after treatment by therapeutic hypothermia of the present invention, the effectiveness of therapeutic hypothermia on a nHIE-affected newborn, who is a subject, can be predicted.

### Brief Description of Drawings

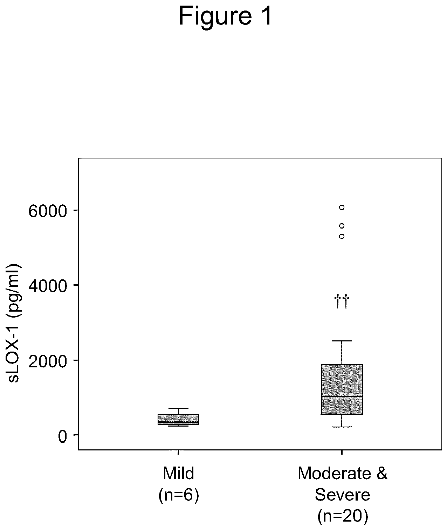

[Figure 1] Figure 1 shows the relationship between the amount of sLOX-1 protein in plasma of nHIE-affected newborn collected within 6 hours after birth and the severity of nHIE. In the figure, O shows a sample with an extremely high amount of sLOX-1 protein. In the figure, †† shows P<0.01 vs mild nHIE.

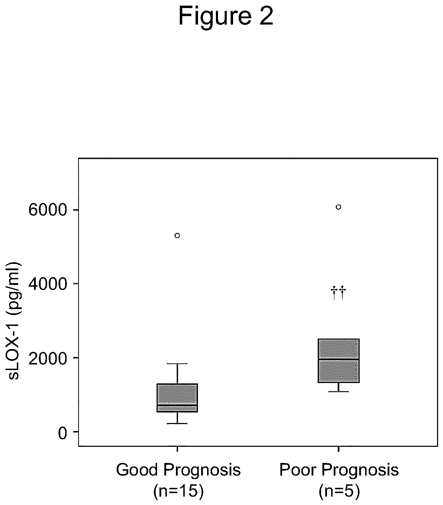

[Figure 2] Figure 2 shows the relationship between the amount sLOX-1 protein in plasma of nHIE-affected newborn collected within 6 hours after birth and the prognosis when the newborns were treated by therapeutic hypothermia. In the figure, O shows a sample with an extremely high amount of sLOX-1 protein. In the figure, †† shows P <0.01 vs good prognosis.

### Description of Embodiments

### 1. Method for determining the severity of neonatal hypoxic-ischemic encephalopathy (method for determining the severity of nHIE)

### 1-1. Overview

A first aspect in this specification is a method for determining the severity of neonatal hypoxic-ischemic encephalopathy (nHIE). This method is capable of determining the severity of nHIE of a subject by measuring the amount of a nHIE marker comprised in blood collected from the subject within a predetermined time.

### 1-2. Definition

The following terms used in this specification are as defined below.

The term "neonatal hypoxic-ischemic encephalopathy (nHIE)" as used herein refers to, as described above, one of cerebrovascular disorders in the newborn, which develops unexpectedly due to severe asphyxia neonatorum, acute respiratory-circulatory failure, fetal hypoxia, or the like.

The term "severity" as used herein refers to the degree of disease symptoms based on an evaluation scale. The term "severity" as used herein refers to the severity of nHIE unless otherwise specified. The severity of nHIE can be, for example, but is not limited to, classified into 4 groups (normal group, mild group [stage 1], moderate group [stage 2], and severe group [stage 4]) based on the nHIE diagnostic criteria of the US NICHD (Shankaran S, et al., 2005, N Engl J Med., 353 (15): 1574-1584), or Sarnat Classification for the severity of nHIE (Sarnat H.B. & Sarnat M.S., 1976, Arch Neurol., 33 (10): 696-705).

The expression "determin(ing) the severity" as used herein refers to identifying the severity of a disease in a subject. The disease in this specification is nHIE, and thus the expression "determining the severity" refers to, specifically identifying the severity of nHIE in a subject.

The term "nHIE marker" as used herein refers to a biomarker for detecting nHIE. Specifically, the nHIE marker as used herein refers to a peptide marker consisting of the LOX-1 protein, particularly, the soluble LOX-1 protein or a fragment thereof.

The term "LOX-1 (lectin-like oxidized low-density lipoprotein receptor-1)" protein refers to a single-transmembrane type membrane protein having the N-terminal side exposed in the cytoplasm and the C-terminal side exposed outside the cytoplasm. The protein forms a homodimer via disulfide bonding and functions as a scavenger receptor for oxidized low-density lipoproteins. The expression of the LOX-1 protein is known to be induced by ischemia-reperfusion injury, active oxygen, and inflammatory cytokines in platelets, endothelial cells, vascular smooth muscle, neurons and macrophages.

A LOX-1 gene encoding the LOX-1 protein can be a biomarker for nHIE detection because its expression is significantly increased with the onset of nHIE (Patent Literature 1). In nHIE patients, with an increase in LOX-1 gene expression, its translation product, the LOX-1 protein, is also significantly increased. Therefore, the LOX-1 protein and a fragment thereof can also be useful biomarkers for detecting nHIE.

The term "LOX-1 protein" as used herein refers to a human LOX-1 protein unless otherwise specified. The LOX-1 protein comprises a wild-type and mutant. The term "mutant" comprises splicing variants and mutants based on SNIPs and the like. The wild-type LOX-1 protein is specifically a polypeptide consisting of the amino acid sequence shown in SEQ ID NO: 1. Further, the mutant LOX-1 protein comprises, for example, but is not limited to, a polypeptide having the amino acid sequence shown in SEQ ID NO: 1 with a deletion(s), substitution(s), or addition(s) of one or plural amino acids, or, a polypeptide having 70% or more, 75% or more, 80% or more, 85% or more, 88% or more, 90% or more, 91% or more, 92% or more, 93% or more, 94% or more, 95% or more, 96% or more, 97% or more, 98% or more or 99% or more amino acid identity with respect to the amino acid sequence shown in SEQ ID NO: 1.

The term "plural" as used herein refers to, for example, 2 to 20, 2 to 15, 2 to 10, 2 to 7, 2 to 5, 2 to 4, or 2 to 3. Further, the term "amino acid identity" refers to the percentage (%) of the number of matched amino acid residues in the total number of amino acid residues when two amino acid sequences to be compared are aligned with appropriately inserting gaps to one or both of them as necessary, so that the number of matched amino acid residues between the two is maximized. Alignment of two amino acid sequences for calculating amino acid identity can be performed using a known program such as Blast, FASTA, or ClustalW.

The term "substitution (of amino acid)" as used herein refers to substitution within conservative amino acid groups having similar properties such as charge, side chain, polarity, and aromaticity among the 20 types of amino acids constituting a natural protein. For example, it comprises substitution within an uncharged polar amino acid group having low-polarity side chains (Gly, Asn, Gln, Ser, Thr, Cys, Tyr), a branched-chain amino acid group (Leu, Val, Ile), a neutral amino acid group (Gly, Ile, Val, Leu, Ala, Met, Pro), a neutral amino acid group having hydrophilic side chains (Asn, Gln, Thr, Ser, Tyr, Cys), an acidic amino acid group (Asp, Glu), a basic amino acid group (Arg, Lys, His) and an aromatic amino acid group (Phe, Tyr, Trp). Amino acid substitution within these groups is preferred because it is known that the properties of a polypeptide are unlikely to be changed with.

The term "soluble LOX-1 protein" (soluble form of the LOX-1 protein: often referred to as the "sLOX-1 protein" in this specification) as used herein refers to a peptide fragment consisting of the extracellular region of the LOX-1 protein. The LOX-1 protein has a protease-sensitive site in the neck domain located on the N-terminal side of the extracellular domain. It is known that when the LOX-1 protein is cleaved at this site, the extracellular region becomes liberated state and released outside the cell and appears in blood (21, 22). In the present invention, since the mass of the LOX-1 protein and/or a fragment thereof present in blood is measured, a nHIE marker that can be a target of the present invention is necessarily the sLOX-1 protein and/or a fragment thereof. The sLOX-1 protein is, in the wild type, a polypeptide consisting of 183 amino acids corresponding to positions 91 to 273 and positions 94 to 273 of the amino acid sequence shown in SEQ ID NO: 1.

The above term "fragment thereof' refers to a fragment of the above sLOX-1 protein. Specifically, it is a polypeptide consisting of successive 6 to 182, 7 to 182, 8 to 182, 9 to 182 or 10 to 182 amino acids comprised in the sLOX-1 protein shown in SEQ ID NO: 2.

The term "subject" as used herein refers to a human individual as a target, more specifically, a newborn applying the method or the apparatus of the present invention. In the method for determining the severity of nHIE, the first aspect of the present invention, and in the inventions of second and the following aspects described later, nHIE patients, that is, nHIE-affected newborns are targeted. Therefore, in principle, subjects to be targeted in all aspects of the present invention are nHIE patients. However, since a nHIE marker to be used in all aspects of the present invention can also detect the presence or the absence of affection with nHIE, it is possible to determine whether the subject is a nHIE patient or not, simultaneously with determination of the severity of nHIE, or the like. Thus, in all aspects of the present invention, a subject may be a newborn who is yet to be determined being affected by nHIE.

### 1-3. Method

The method for determining the severity of nHIE of the present invention comprises a step of obtaining a measurement value, and a step of determining the severity as essential steps. Each step is specifically described as follows.

### 1-3-1. Step of obtaining measurement value

The "step of obtaining a measurement value" is a step of measuring the mass per unit volume of a nHIE marker comprised in blood, so as to obtain the measurement value.

Blood to be subjected to measurement in this step is blood collected from a subject within a predetermined time after birth. The term "within a predetermined time" refers to within 6 hours, within 5 hours, within 4 hours, within 3 hours, within 2 hours, within 1 hour, within 30 minutes, or immediately (within 5 minutes) after birth. Further, blood to be subjected to measurement in this step may be umbilical artery blood (umbilical artery blood at birth) flowing from the fetus to mother among umbilical cord blood obtained at birth. "Blood" as used herein refers to whole blood, serum, or plasma. Any blood may be used, but measurement of the sLOX-1 protein is easily affected by blood coagulation, hemolysis, interstitial fluid contamination, and the like. Therefore, preferred blood is plasma that is less susceptible thereto and allows accurate quantification of the sLOX-1 protein.

A nHIE marker to be measured in this step is the sLOX-1 protein and/or a fragment thereof that can be present in blood as described above. The value to be measured is the mass of the nHIE marker present in blood per unit volume. The volume of blood to be subjected to measurement in this step may be, for example but is not limited to, a blood volume ranging from 1µL to 10µL, 2 µL to 20 µL, 5µL to 50 µL, 8 µL to 80 µL, 10 µL to 100 µL, 20 µL to 200 µL, 50 µL to 500 µL, 80 µL to 800 µL, 100 µL to 1 mL, 200 µL to 2 mL or 500 µL to 5 mL.

The method for measuring the mass of the nHIE marker in blood is not particularly limited as long as it is a peptide quantification method. For example, a known quantification method such as an immunological detection method, an aptamer analysis method, or a mass spectrometry method can be used. Hereinafter, each quantification method will be described.

### (1) Immunological detection method

The "immunological detection method" is a method for quantifying a target molecule using an antibody or a fragment thereof that specifically binds to the target molecule. The immunological detection method comprises enzyme immunoassay (including ELISA and EIA), fluorescence immunoassay, radioimmunoassay (RIA), luminescence immunoassay, a surface plasmon resonance method (SPR method), a quartz crystal microbalance (QCM) method, an immunoturbidimetric method, latex agglutination immunoassay, latex nephelometry, erythrocyte agglutination reaction, a particle agglutination reaction method, a colloidal gold method, capillary electrophoresis, Western blotting or an immunohistochemical method (immunostaining method) and any of these methods may be used.

In this step, since the nHIE marker can be the target molecule, the antibody to be used may be any antibody that can recognize and bind to the extracellular domain of LOX-1. For example, it comprises an anti-LOX-1 antibody and an anti-sLOX-1 antibody. The anti-LOX-1 antibody or the anti-sLOX-1 antibody may be prepared by a conventional method using the full length of the sLOX-1 protein or a portion thereof (for example, the entire or a portion of the extracellular domain) as an antigen. A commercially available anti-LOX-1 antibody or anti-sLOX-1 antibody can also be used. As the antibody, either a monoclonal antibody or a polyclonal antibody may be used. When the antibody is a polyclonal antibody or a monoclonal antibody, an immunoglobulin constituting the antibody can be of any class (e.g., IgG, IgE, IgM, IgA, IgD and IgY), or of any subclass (eg, IgG1, IgG2, IgG3, IgG4, IgA1, IgA2). The antibody may be derived from any animal including mammals and birds. Animals from which the antibody is derived comprise, for example, rabbits, mice, rats, guinea pigs, goats, donkeys, sheep, camels, llamas, alpaca, horses, chickens, and humans, and the like.

In the immunological detection method, an artificially prepared antibody based on a polyclonal antibody or a monoclonal antibody can also be used. Such an antibody comprises, for example, a recombinant antibody, synthetic antibody, and antibody fragment.

The "recombinant antibody" is an antibody prepared using genetic recombination techniques, and comprises chimeric antibodies, humanized antibodies, and multi-specific antibodies, and the like. The "chimeric antibody" is an antibody in which a variable region (V region) of an antibody is substituted with a V region of an antibody derived from another animal using molecular biological techniques. For example, it comprises an antibody having the V region derived from a mouse and the C region derived from a human, in which a V region of the human antibody is substituted with the V region of a mouse anti-sLOX-1 monoclonal antibody that specifically binds to a nHIE marker. The "humanized antibody" is a graft antibody in which complementarity determining regions (CDRs: CDR1, CDR2, and CDR3) in a V region of a non-human mammal, for example, a mouse anti-sLOX-1 monoclonal antibody or a mouse anti-sLOX-1 monoclonal antibody that specifically binds to a nHIE marker is substituted with the CDRs of a human monoclonal antibody. The "multi-specific antibody" is a multivalent antibody, that is, an antibody having plural antigen-binding sites in one molecule, in which each of the antigen-binding sites binds to different epitope. For example, for an antibody having two antigen-binding sites such as IgG, an bispecific antibody is exemplified, in which each of the antigen-binding sites specifically binds to the same or different nHIE marker.

The "synthetic antibody" is an antibody synthesized by using a chemical method or a recombinant DNA method. It comprises a monomeric polypeptide molecule, in which one or more light chain variable regions (VL regions) and one or more heavy chain variable regions (VH regions) of a specific antibody are artificially linked via linker peptides or the like having appropriate lengths and sequences, and multimeric polypeptides thereof. A specific example of such polypeptides comprises a single chain Fv (scFv: single chain Fragment of variable region) (see Pierce Catalog and Handbook, 1994-1995, Pierce Chemical Co., Rockford, IL), diabody, triabody or tetrabody, or the like. In immunoglobulin molecules, VL and VH regions are usually located on separate polypeptide chains (light chain and heavy chain). Single-chain Fv is a synthetic antibody fragment having a structure in which V regions on these two polypeptide chains are linked by a flexible linker having a sufficient length, so as to be comprised in a single polypeptide chain. Within a single chain Fv, both V regions can self-assemble with each other to form one functional antigen binding site. Single-chain Fv can be obtained by incorporating and expressing a recombinant DNA encoding it in a phage genome using known techniques. A diabody is a molecule having a structure based on the dimeric structure of single-chain Fv (Holliger et al., 1993, Proc. Natl. Acad. Sci. USA 90: 6444-6448). For example, if the length of the linker is shorter than about 12 amino acid residues, the two variable sites within the single-chain Fv cannot self-assemble. However, the formation of a diabody, i.e., the interaction between two single-chain Fvs enable the VL region of one Fv chain to assemble with the VH region of the other Fv chain and to form two functional antigen binding sites (Marvin et al., 2005, Acta Pharmacol. Sin. 26: 649-658). Furthermore, the addition of a cysteine residue to the C-terminus of the single-chain Fv enables disulfide bonding between the two Fv chains and to form a stable diabody (Olafsen et al., 2004, Prot. Engr. Des. Sel. 17: 21-27). As described above, a diabody is a bivalent antibody fragment, but each of the antigen-binding sites does not need to bind to the same epitope, and may have bispecificity each recognizing and specifically binding to different epitopes. Triabody and tetrabody are based on a single-chain Fv structure and have its trimeric and tetrameric structures, as in a diabody. These are trivalent and tetravalent antibody fragments, respectively, and may be multi-specific antibodies.

The "antibody fragment" comprises Fab, F (ab'₂), and Fv.

### (2) Aptamer analysis method

The "aptamer analysis method" is a method for quantifying a nHIE marker as a target molecule using an aptamer that binds firmly and specifically to a target substance because of the steric structure. The aptamer can be roughly classified into nucleic acid aptamer and peptide aptamer depending on the type of the molecule, and may be any aptamer.

The "nucleic acid aptamer" refers to an aptamer constituted with a nucleic acid. The nucleic acid constituting a nucleic acid aptamer may be any of DNA, RNA, or a combination thereof. A chemically modified nucleic acid such as PNA, LNA/BNA, methylphosphonate DNA, phosphorothioate DNA, and 2' -O-methyl RNA can also be comprised, as necessary.

Nucleic acid aptamers can be prepared by a method known in the art such as a SELEX (systematic evolution of ligands by exponential enrichment) method, using a nHIE marker, that is, the full length of or a portion of the sLOX-1 protein as a target molecule. The SELEX method is a known method, and the specific method can be performed according to, for example, Pan et al. (Proc. Natl. Acad. Sci. 1995, U.S.A.92: 11509-11513).

A peptide aptamer is an aptamer constituted with amino acids, and is a 1-6 kD peptide molecule that recognizes and binds specifically to the surface structure of a specific target molecule as that of an antibody. It can be produced using a phage display method or a cell surface display method. For a method for producing a peptide aptamer, it can be prepared based on a method known in the art. For example, reference can be made to Whaley, S.R., et al., 2000, Nature, 405, 665-668.

The above antibody or aptamer may be labeled as necessary. For labeling, a labeling substance known in the art may be used. In the case of antibody and peptide aptamer, for example, labeling can be performed with a fluorescent dye (fluorescein, FITC, rhodamine, Texas red, Cy3, Cy5), fluorescent protein (e.g., PE, APC, GFP), enzyme (e.g., horseradish peroxidase, alkaline phosphatase, glucose oxidase), radioisotope (e.g. ³H, ¹⁴C, ³⁵S) or biotin or (strept)avidin. Also for a nucleic acid aptamer, an example of labeling substance comprises a radioisotope (e.g., ³²P, ³H, ¹⁴C), DIG, biotin, fluorescent dye (e.g., FITC, Texas, cy3, cy5, cy7, FAM, HEX, VIC, JOE, Rox, TET, Bodipy493, NBD, TAMRA), or a luminescent substance (for example, acridinium ester). An antibody or aptamer labeled with a labeling substance can be a useful tool upon detecting an aptamer bound to a target protein.

### (3) Mass spectrometry

"Mass spectrometry" may comprise high performance liquid chromatography mass spectrometry (LC-MS), high performance liquid chromatography tandem mass spectrometry (LC-MS/MS), gas chromatography mass spectrometry (GC-MS), gas chromatograph tandem mass spectrometry (GC-MS/MS), capillary electrophoresis mass spectrometry (CE-MS) and ICP mass spectrometry (ICP-MS).

The above immunological detection method, aptamer analysis method, and mass spectrometry method are all techniques known in the art, and may be performed according to these methods. For example, these techniques can be performed according to methods described in Green, M.R. and Sambrook, J., 2012, Molecular Cloning: A Laboratory Manual Fourth Ed., Cold Spring Harbor Laboratory Press, Cold Spring Harbor, New York; Christopher J., et al., 2005, Chemical Review,105:1103-1169;Iijima Y. et al., 2008,. The Plant Journal, 54,949-962;Hirai M. et al.,2004, Proc Natl Acad Sci USA, 101(27) 10205-10210;Sato S, et al., 200fourthe Plant Journal, 40(1)151-163; Shimizu M. et al., 2005, Proteomics, 5,3919-3931. Furthermore, various peptide quantification kits are commercially available from various manufacturers, and they can also be used herein.

### 1-3-2. Step of determining severity

The "step of determining severity" is a step of assisting determination of the severity of nHIE based on a measurement value obtained in the above step of obtaining a measurement value. This step involves defining a predetermined value among the measurement values as a cut-off value, and determining that the severity of nHIE in a subject who is the donor of blood used in the step of obtaining a measurement value is moderate or more; that is, moderate or severe, if the measurement value is the cut-off value or more.

The above cut-off value is, specifically, for example, 310 pg/mL, preferably 320 pg/mL, more preferably 330 pg/mL, and further preferably 335 pg/mL. On the other hand, when a measurement value is less than the cut-off value, the severity of nHIE of a subject who is the donor of blood used in the above step of obtaining a measurement value can also be determined to be mild.

### 1-4. Effect

According to the method for determining the severity of nHIE of the present invention, the use of blood of a subject (nHIE patient) collected within a predetermined time after birth can assist doctors in diagnosing, in determining the severity of the subject affected with nHIE, with high specificity and predictive value. Further, the method enables more convenient and objective determination based on the amount of nHIE marker in blood, and thus can be a new evaluation scale of the severity of nHIE in place of the conventional nHIE diagnostic criteria of the US NICHD and Sarnat Classification for the severity of nHIE.

### 2. Method for determining the necessity of the application of therapeutic hypothermia

### 2-1. Outline

A second aspect in this specification is a method for determining the necessity of the application of therapeutic hypothermia to a nHIE patient. This method predicts based on a measurement value of the amount of a nHIE marker comprised in blood collected from a subject within a predetermined time after birth. According to the method for determining the application of therapeutic hypothermia of the present invention, it is possible to easily determine whether or not the subject needs the application of therapeutic hypothermia thereto.

### 2-2. Method

A method for applying therapeutic hypothermia of the present invention comprises a step of obtaining a measurement value, and a step of determining the application of therapeutic hypothermia as essential steps. Herein after, each step is specifically described.

### 2-2-1. Step of obtaining measurement value

The "step of obtaining a measurement value" is a step of measuring the mass per unit volume of a nHIE marker comprised in blood, so as to obtain the measurement value. The step of obtaining a measurement value in this aspect is the same as the step of obtaining a measurement value in the method for determining the severity of nHIE of the first aspect. Therefore, the specific description is omitted here.

### 2-2-2. Step of determining the application of therapeutic hypothermia

The "step of determining the necessity of the application of therapeutic hypothermia" is a step of assisting the determination whether or not to apply therapeutic hypothermia to a subject based on a measurement value obtained in the above step of obtaining a measurement value. The basic constitution of this step is the same as that of the step of determining the severity in the method for determining the severity of nHIE of the first aspect. The step of determining severity defines a predetermined measurement value as a cut-off value, and determining that the severity of nHIE in the subject is moderate or more, if a measurement value is the cut-off value or more, whereas this step defines the same measurement value as in the step of determining severity as a cut-off value, and determines that the subject needs the application of therapeutic hypothermia, if a measurement value is the cut-off value or more. That is, when the severity of a subject is determined to be moderate or more in the step of determining severity of the first aspect, this step of this aspect determines that the subject needs the application of therapeutic hypothermia.

On the other hand, when a measurement value is less than the cut-off value, a subject who is the donor of blood used in the above step of obtaining a measurement value can also be determined not to need therapeutic hypothermia. This is because if a measurement value is less than the cut-off value, the subject's nHIE severity is mild based on the method for determining the severity of nHIE of the first aspect.

### 2-3. Effect

According to the method for determining the necessity of the application of therapeutic hypothermia of the present invention, the use of the blood of a subject (nHIE patient) collected within a predetermined time after birth can assist doctors in diagnosing by determining whether or not the subject is a nHIE patient to whom therapeutic hypothermia should be applied, with high specificity and predictive value.

### 3. Method for predicting prognosis after treatment by therapeutic hypothermia

### 3-1. Outline

A third aspect in this specification is a method for predicting the prognosis of a nHIE patient, when the patient is treated by therapeutic hypothermia. This method predicts the prognosis when a subject is treated by therapeutic hypothermia based on the measurement value of the amount of a nHIE marker comprised in blood collected from the subject within a predetermined time after birth. According to the method for predicting the prognosis after treatment, by therapeutic hypothermia of the present invention, the effectiveness of therapeutic hypothermia for a subject can be predicted.

### 3-2. Method

The method for applying therapeutic hypothermia of the present invention comprises a step of obtaining a measurement value, and a step of predicting the prognosis as essential steps. Herein after, each step is specifically described.

### 3-2-1. Step of obtaining measurement value

The "step of obtaining a measurement value" is a step of measuring the mass per unit volume of a nHIE marker comprised in blood, so as to obtain the measurement value. The step of obtaining a measurement value of this aspect is basically the same as the step of obtaining a measurement value in the method for determining the severity of nHIE of the first aspect. Therefore, only characteristic features of the step of obtaining a measurement value in this aspect will be described here.

A subject who is a target of the present invention is a nHIE-affected newborn. Blood to be used in this step is, as described in the step of obtaining a measurement value of the first aspect, blood collected within a predetermined time after birth of a subject. At the time of blood collection, a subject may have already been treated by therapeutic hypothermia or may be before the treatment. The method for predicting the prognosis after treatment by therapeutic hypothermia of this aspect can predict the prognosis after treatment, regardless of before or after the start of therapeutic hypothermia for a subject. In view of the fact that the present invention is a method for predicting the effectiveness of therapeutic hypothermia for a subject, a subject before the treatment is preferably a target. This is because when poor prognosis is predicted even with treatment by therapeutic hypothermia, a determination can be made such as selecting other effective nHIE therapeutic methods other than hypothermia, or using therapeutic hypothermia and other effective nHIE therapeutic methods in combination.

### 3-2-2. Step of predicting prognosis

The "step of predicting the prognosis" is a step of predicting whether the prognosis of a subject before or after treatment by therapeutic hypothermia is good or not, based on a measurement value obtained in the above step of obtaining a measurement value.

This step involves defining a predetermined measurement value as a cut-off value as in the step of determining the severity of the first aspect and the step of determining the application of therapeutic hypothermia of the second aspect, and then predicting the prognosis based on the cut-off value. Specifically, if a measurement value is less than the cut-off value, the prognosis of the above subject after treatment by therapeutic hypothermia is predicted to be good.

The cut-off value in this step differs from the cut-off values defined in the first and the second aspects. Specifically, it is, for example, 1400 pg/mL, preferably 1500 pg/mL, more preferably 1600 pg/mL, and further preferably 1650 pg/mL. As described in the following Example, when a measurement value is less than 1610 pg/mL, the prognosis of a subject treated by therapeutic hypothermia is good with specificity of 60% and a positive predictive value of about 86%.

On the other hand, when a measurement value is equal to or higher than the above cut-off value, the subject is likely to have a poor prognosis even if treated by therapeutic hypothermia. Therefore, if therapeutic hypothermia will be performed or after being performed, continuous observation after discharge and immediate response system in an emergency or performing other effective therapeutic methods should be examined in advance.

### 3-3. Effect

According to the method for predicting the prognosis after treatment by therapeutic hypothermia of the present invention, the use of blood of a subject (nHIE patient) collected within a predetermined time after birth enables to predict whether the later prognosis concerning nHIE after that is good or not, when the subject is treated or has already been treated by therapeutic hypothermia.

### 4. Apparatus for determining the severity of neonatal hypoxic-ischemic encephalopathy (apparatus for determining the severity of nHIE)

### 4-1. Outline

A fourth aspect not part of the present invention is an apparatus for determining the severity of neonatal hypoxic-ischemic encephalopathy (apparatus for determining the severity of nHIE; device for determining the severity of nHIE). The apparatus for determining the severity can, by using blood collected from a subject within a predetermined time after birth, determine the severity of nHIE of the subject conveniently and with a high predictive value. Therefore, the apparatus can provide auxiliary information useful for doctors to diagnose the severity of nHIE patients.

### 4-2. Constitution

The apparatus for determining the severity of nHIE comprises a blood receiving part, a reagent part, a reaction part, and a presenting part as essential constituent elements, and a spreading part as an optional constituent element. The spreading part can further comprise a labeling means. Herein after, each constituent element is described. For the apparatus for determining the severity a test object is, but is not limited to, blood collected from one subject with single collection.

### 4-2-1. Blood receiving part

The "blood receiving part" is constituted to receive blood collected from a subject. Blood received in this part is preferably, but is not limited to, blood collected from a subject within a predetermined time after birth of the subject, for example, within 6 hours after birth. The amount of blood to be received is not limited as long as it is the minimum amount required for determining the severity of nHIE patients or more. It depends on the sensitivity of a sLOX-1 detection reagent comprised in the reagent part, but it may usually be 50 µL or more, 100 µL or more, 200 µL or more, 500 µL or more, 800 µL or more, or 1.0 mL or more, and 1.5 mL or less, 2.0 mL or less, 3.0 mL or less, 4.0 mL or less, or 5.0 mL or less.

The blood receiving part may have a well-like container shape capable of storing the received blood. In this case, the material of the blood receiving part is not limited as long as it is constituted with a material that does not denature blood or is not denatured by blood. It comprises a material such as a plastic including polypropylene and polystyrene, and the like, glass, or paper with its surface being specially coated. Further, the blood receiving part may have a sheet-like or a rod-like shape that easily absorbs blood, which is a target sample. In this case, the blood receiving part may be constituted with a material that absorbs blood, which is a target sample, but does not denature blood or is not denatured by blood, such as a nonwoven fabric or a filter.

When the severity of nHIE is determined using the apparatus for determining the severity of nHIE, blood to be tested may be dropped or impregnated into the blood receiving part.

### 4-2-2. Reagent part

The "reagent part" is constituted to comprise a sLOX-1 detection reagent. The "sLOX-1 detection reagent" is a reagent capable of detecting the sLOX-1 protein or a fragment thereof (often referred to as "the sLOX-1 protein and the like." in this specification) that is a nHIE marker, and comprises a substance capable of specifically recognizing and binding to a LOX-1 extracellular domain and the sLOX-1 protein and the like. Specifically, for example, various detection agents used in the method for measuring the mass of the nHIE marker in blood in the step of obtaining a measurement value of the method for determining the severity of nHIE of the first aspect is comprised. A specific example thereof comprises an anti-LOX-1 antibody or anti-sLOX-1 antibody or fragment thereof, a chemically modified derivative thereof, or a LOX-1 aptamer or a sLOX-1 aptamer that binds to the extracellular domain of LOX-1. The sLOX-1 detection reagent may be immobilized on the surface of a carrier in the reagent part, or may be in a free state within the reagent part.

The amount of the sLOX-1 detection reagent comprised in the reagent part is not limited as long as the amount of a nHIE marker comprised in blood can be detected at least at the minimum amount required for determining the severity of nHIE patients.

The reagent part may be installed at a position different from the blood receiving part in the apparatus for determining the severity of nHIE. In this case, the reagent part and the blood receiving part are constituted such that blood received by the blood receiving part and the sLOX-1 detection reagent comprised in the reagent part are delivered respectively to the reaction part described later through flow paths. In addition, the blood receiving part and the reagent part can be installed at the same position to form a blood receiving and reagent part (blood receiving part/reagent part). In this case, the sLOX-1 detection reagent is comprised in the blood receiving part, and the received blood reacts immediately with the sLOX-1 detection reagent. Therefore, the blood receiving part/reagent part can also serve as a reaction part described below.

### 4-2-3. Reaction part

The "reaction part" is constituted with function as a field where the sLOX-1 protein and the like comprised in the above blood can react with a sLOX-1 detection reagent. The constitution is not particularly limited as long as it can provide conditions under which the reaction of the two can proceed. For example, if the sLOX-1 detection reagent is an anti-sLOX-1 antibody, the reaction part may be constituted to be able to function as a field with conditions where the anti-sLOX-1 antibody can bind to the sLOX-1 protein and the like comprised in blood received, and form an antigen-antibody complex.

### 4-2-4. Spreading part

The "spreading part" is a part that can be a flow path through which a reaction product generated in the reaction part migrates to the presenting part described later, and is a selective constituent element in the apparatus for determining of the present invention. The spreading part can function as a part for connecting both reaction and presenting parts, when these parts are installed at different positions in the apparatus. The structure of the spreading part is not limited, but comprises a groove-like or tubular flow path.

### (1) Labeling means

The spreading part may comprise a labeling means. The "labeling means" is a means for labeling a reaction product until the reaction product generated in the reaction part reaches the presenting part. The labeling means is constituted such that a reaction product is labeled with a labeling substance. The specific constitution of the labeling means is not limited, but, for example, the labelling means may be constituted such that complexes formed in the reaction part by binding of the sLOX-1 protein and the like in blood and a sLOX-1 detection reagent are labeled with a labeled secondary antibody or the like specifically binding to the sLOX-1 detection reagent or the sLOX-1 protein and the like in the complexes.

Any substance that is common in the art may be used as a labeling substance, and is not limited. For example, labeling by fluorescent dyes (including such as FITC, rhodamine, Texas red, Cy3, Cy5), fluorescent proteins (including such as PE, APC, GFP, EGFP), enzymes (including such as horseradish peroxidase, alkaline phosphatase, glucose oxidase), or biotin, avidin, streptavidin, and the like may be comprised.

In addition, the labeling means may be comprised in the reaction part or the presenting part described later.

### 4-2-5. Presenting part

The "presenting part" is constituted to be able to present the severity of nHIE in the above subject based on the detection result in the reaction part, that is, the amount of the reaction product generated in the reaction part. Specifically, the presenting part may be constituted to be able to present that the severity of nHIE is moderate or more, such as in the form of positive reaction, when the amount of the sLOX-1 protein and the like comprised in blood is 330 pg/mL or more. Conversely, the presenting part may also be constituted to be able to present that the severity of nHIE is mild, such as in the form of negative reaction, when the amount of the sLOX-1 protein and the like comprised in blood is less than 330 pg/mL.

Any presenting means may be employed. For example, when the apparatus for determining the severity of nHIE comprises the above labeling means, it may be constituted to be able to present color developed if a complex is colored by the labeling substance, or to present fluorescence or luminescence if the labeling substance is a fluorescent substance or a luminescent substance, at any location on the apparatus for determining the severity of nHIE.

As a specific example of the apparatus for determining the severity of nHIE of this aspect, an immunochromatographic test strip known in the art is comprised. Commercially available pregnancy diagnostic agents and the like have the similar form and principle as the apparatus. An immunochromatographic test strip has extremely low invasiveness and do not pose any pain or danger due to the use of the reagent to a subject. Further, a test strip is also preferable in that they can be mass-produced at a low cost.

### 5. Apparatus for determining the necessity of applying therapeutic hypothermia

### 5-1. Outline

A fifth aspect not part of the present invention is an apparatus for determining the necessity of applying therapeutic hypothermia (device for determining the necessity of applying therapeutic hypothermia). The apparatus uses blood collected from a subject within a predetermined time after birth, so that whether or not the subject who is a nHIE patient needs the application of therapeutic hypothermia can be determined conveniently with a high predictive value. Therefore, the apparatus can provide auxiliary information useful for a doctor to diagnose whether or not a subject should be treated by therapeutic hypothermia.

### 5-2. Constitution

The apparatus for determining the necessity of applying therapeutic hypothermia comprises a blood receiving part, a reagent part, a reaction part, and a presenting part as essential constituent elements, and a spreading part as an optional constituent element. The spreading part can further comprise a labeling means. These constitutions are almost the same as those of the respective parts of the apparatus for determining the severity of nHIE of the fourth aspect. Therefore, descriptions for the parts (blood receiving part, reagent part, reaction part, and spreading part) and means (labeling means) having the constitutions shared by both apparatuses are omitted, and only the presenting part having a constitution partially different from that of the apparatus for determining the severity of nHIE is described herein after.

### 5-2-1. Presenting part

The presenting part of the apparatus for determining the necessity of applying therapeutic hypothermia of this aspect has the same basic constitution as that of the presenting part of the above apparatus for determining the severity of nHIE. However, the information to present is different from that presented by the apparatus for determining the severity of nHIE. That is, although the presenting part of the apparatus for determining the severity of nHIE is constituted to be able to present the severity of nHIE in a subject, the presenting part of the apparatus for determining the necessity of applying therapeutic hypothermia of this aspect is constituted to be able to present the necessity of therapeutic hypothermia for a subject.

Specifically, the presenting part may be constituted to be able to present that a subject who donated blood requires the application of therapeutic hypothermia, such as in the form of positive reaction, when the amount of the sLOX-1 protein and the like comprised in the blood is 330 pg/mL or more.

Conversely, the presenting part may also be constituted to be able to present that a subject who donated blood requires no application of therapeutic hypothermia, such as in the form of negative reaction, when the amount of the sLOX-1 protein and the like, comprised in the blood is less than 330 pg/mL.

Any presenting means can be employed. For example, when the apparatus for determining the necessity of applying therapeutic hypothermia comprises the above labeling means, it may be constituted to be able to present color developed if a complex is colored by a labeling substance, or to present fluorescence or luminescence if the labeling substance is a fluorescent substance or a luminescent substance, at any location on the apparatus for determining the necessity of applying therapeutic hypothermia.

### 6. Apparatus for predicting prognosis after treatment by therapeutic hypothermia

### 6-1. Outline

The sixth aspect in this specification but not part of the invention is an apparatus for predicting the prognosis after treatment by therapeutic hypothermia. The apparatus for predicting the prognosis after treatment by therapeutic hypothermia of this aspect uses blood collected from a subject within a predetermined time after birth, so as to be able to predict the prognosis of the subject who is a nHIE patient conveniently and with a high predictive value, when they are treated by therapeutic hypothermia. Therefore, based on the prediction results of the apparatus for predicting the prognosis after treatment by therapeutic hypothermia of this aspect, information concerning the necessity of applying therapeutic hypothermia and, even after the treatment, the necessity of continuous observation after discharge and immediate response system in an emergency can be provided to doctors.

### 6-2. Constitution

The apparatus for predicting the prognosis after treatment by therapeutic hypothermia comprises a blood receiving part, a reagent part, a reaction part, and a presenting part as essential constituent elements, and a spreading part as an optional constituent element. The spreading part can further comprise a labeling means. These constitutions are almost the same as those of the respective parts of the apparatus for determining the severity of nHIE of the fourth aspect. Therefore, the descriptions of the parts (blood receiving part, reagent part, reaction part, and spreading part) and means (labeling means) having the constitutions shared by both apparatuses are omitted, and only the presenting part having a constitution partially different from that of the apparatus for determining the severity of nHIE is described herein after.

### 6-2-1. Presenting part

The presenting part of the apparatus for predicting the prognosis after treatment by therapeutic hypothermia of this aspect has the same basic constitution as that of the presenting part of the above apparatus for determining the severity of nHIE. However, the information to present is different from that presented by the apparatus for determining the severity of nHIE. That is, although the presenting part of the apparatus for determining the severity of nHIE is constituted to be able to present the severity of nHIE in a subject, the presenting part of the apparatus for predicting the prognosis after treatment by therapeutic hypothermia of this aspect is constituted to be able to present prediction of the prognosis after treatment of a subject by therapeutic hypothermia.

Specifically, the presenting part may be constituted to be able to present that the prognosis of a subject who donated blood is good if the subject is treated by therapeutic hypothermia, such as in the form of negative reaction, when the amount of the sLOX-1 protein and the like comprised in blood is less than1650 pg/mL.

Conversely, the presenting part may be constituted to be able to present that the prognosis of a subject who donated blood is poor, even if the subject is treated by therapeutic hypothermia, such as in the form of positive reaction, when the amount of the sLOX-1 protein and the like comprised in blood is 1650 pg/mL or more. In this case, employment of other effective nHIE therapeutic methods other than therapeutic hypothermia, or using of therapeutic hypothermia with other effective nHIE therapeutic methods in combination can be examined. Further, even when the subject has been treated by therapeutic hypothermia, understanding that signs of improvement of the subject is limited, preparation in advance such as the followup of the subject after discharge and establishing the immediate response system at the time of abnormal onset due to recurrence is made possible.

Any presenting means can be employed. For example, when the apparatus for predicting the prognosis after treatment by therapeutic hypothermia comprises the above labeling means, it may be constituted to be able to present color developed if a complex is colored by a labeling substance, or to present fluorescence and luminescence if a labeling substance is a fluorescent substance or a luminescent substance, at any location on the apparatus for predicting the prognosis after treatment by therapeutic hypothermia.

Described herein but not part of the invention is a kit comprising any one of, or a combination of the apparatuses according to the fourth to the sixth aspects. In this case, the kit of this aspect may also comprise, other than the above apparatus of each aspect, a substrate necessary for the detection of labels, carrier, washing buffers, sample diluents, enzyme substrates, reaction stop solutions, labeled secondary antibodies, and the instructions for use, and the like.

### Examples

### <Method>

### (1) Obtainment of blood sample

Blood samples were collected from newborns hospitalized in the neonatal intensivecare units (NICU) of cooperative facilities (Saitama Children's Medical Center, Tokyo Metropolitan Children's Medical Center, Ome Municipal General Hospital, and The University of Tokyo Hospital) during a period from June 2014 to May 2017. The requirements for newborns to be subjected to blood collection are a gestation period of 36 weeks or more, a birth weight of 1800 g or more, and no abnormalities such as chromosomal abnormalities, congenital infections, congenital heart diseases, and malformations. Blood was collected from 72 newborns who met these requirements. Informed consent was obtained from the parents of the newborns for the use of these blood samples. All experiments conducted in the Examples were approved by the Ethics Committees of the National Center of Neurology and Psychiatry, a National Research and development agency, and of the cooperative facilities.

### (2) Blood collection and measurement of the amount of plasma sLOX-1 protein

At the time of NICU admission, 0.5 mL each of blood was collected from the veins or arteries of the 72 newborns using EDTA-2K tubes. Immediately after blood collection, blood was centrifuged at 3000 rpm for 15 minutes at 4°C, and the supernatant was obtained as a plasma sample. The plasma samples were stored at -80°C until the measurement. Blood sampling was performed at 0, 1, 2 to 4 and 5 to 9 days after birth.

The amount of the sLOX-1 protein in the plasma sample was measured by sandwich ELISA (outsourced) using 2 types of anti-sLOX-1 monoclonal antibodies, according to the method described by Inoue N, et al. (Clin Chem., 2010, 56 (4): 550-558). The measurement results were evaluated with the severity obtained from clinical information, symptoms at discharge, and test data.

### (3) Subject's clinical profile and evaluation

For the diagnosis and the severity of nHIE, the nHIE diagnostic criteria of the US NICHD (1. Strong acidosis found from umbilical cord blood or blood collected within 60 minutes after birth (the result of blood gas analysis: pH 7.0 or lower, or base excess: 16 mmol/L or more); 2. In addition to acute perinatal events (such as placenta detachment), either APGAR score (after 10 minutes) of 5 or less, or resuscitation (assisted ventilation) for 10 minutes or more after birth, was confirmed) was used, and 72 newborns as subjects for analysis were classified into 4 groups: normal group, nHIE mild group, nHIE moderate group, and nHIE severe group according to Modified Sarnat Classification for the severity of nHIE. Specifically, neurological states in six categories: consciousness level, spontaneous activity, posture, muscle tone, primitive reflexes (e.g., sucking and Moro reflexes), and autonomic nervous system functions (e.g., pupils and heart rate) were examined. Newborns with abnormalities in 3 or more categories were classified as newborns with moderate nHIE or severe nHIE, and newborns with abnormalities in 2 or less categories were classified as newborns with mild nHIE. Moderate and severe cases were classified according to the number of signs, and when the distributions of signs were the same, distinguished based on the consciousness levels.

As a result, the details of each group were 45 subjects of the normal group, 6 subjects of the nHIE mild group, 16 subjects of the nHIE moderate group, and 5 subjects of the nHIE severe group. Of 45 subjects of the normal group, 38 subjects from whom samples had been obtained within 6 hours after birth were used as controls.

In order to evaluate the results at discharge, eating disorders, hearing disorders, seizures and paralysis were defined to indicate severe cases. Eating disorders were defined to indicate cases requiring tube feeding, and hearing disorders were defined to indicate cases with no response or weak response to an 80-dB auditory brainstem response.

### (4) Statistical analysis

Statistical analysis software (SPSS Statistics ver.24.0; IBM) was used for statistical analysis. Comparison of the amount of the sLOX-1 protein was performed between the mild nHIE group (therapeutic hypothermia non-adaptative group) and the moderate/severe nHIE group (therapeutic hypothermia adaptative group) using the Mann-Whitney test, and appropriate cut-off values for distinguishing between mild nHIE and moderate/severe nHIE are set by constructing receiver operating characteristic (ROC) curves and calculating the area under a curve (AUC). Cut-off values were set to be 534 pg/mL, 382 pg/mL, and 334 pg/mL. Thus, the subjects were classified into positive subjects exhibiting the amount of the plasma sLOX-1 protein with each cut-off value or more and negative subjects exhibiting the amount less than the cut-off value.

A positive predictive value (PPV) and a negative predictive value (NPV) were calculated using the sensitivity and the specificity calculated from the incidence and the ROC curve of the disease. Specifically, the percentage of moderate/severe nHIE patients in the subjects determined to be positive based on the cut-off values was calculated as the positive predictive value, and the percentage of mild nHIE patients in the subjects determined to be negative was calculated as the negative predictive value.

Subsequently, newborns were compared for prognosis (outcome) at discharge. An ROC curve was constructed to test the performance of the sLOX-1 protein as a prognostic marker at discharge, and, as described above, sensitivity and specificity, a positive predictive value, and a negative predictive value were exemplified. Cut-off values were set to be 1630 pg/mL, 1620 pg/mL, and 1500 pg/mL, the subjects were classified into positive subjects exhibiting the amount of the plasma sLOX-1 protein less than each cut-off value, and negative subjects exhibiting the amount with the cut-off value or more.

Regarding prediction of good prognosis, the percentage of nHIE patients who had good prognosis among subjects determined to be positive based on the cut-off values was calculated as the positive predictive value, and the percentage of nHIE patients who had poor prognosis among subjects determined to be negative was calculated as the negative predictive value.

### <Results>

### (Severity and determination results)

Tables 1 to 3 and Figure 1 show the results.

**[Table 1]**

| sLOX-1≥534 pg/mL | | | |
|---|---|---|---|
| | MODERATE · SEVERE | MILD | PREDICTIVE VALUE |
| POSITIVE | 16 | 2 | 88.9% |
| NEGATIVE | 4 | 4 | 50.0% |
| | SENSITIVITY 80.0% | SPECIFICITY 66.7% | |

**[Table 2]**

| sLOX-1≥382 pg/mL | | | |
|---|---|---|---|
| | MODERATE· SEVERE | MILD | PREDICTIVE VALUE |
| POSITIVE | 17 | 2 | 89.5% |
| NEGATIVE | 3 | 4 | 57.1% |
| | SENSITIVITY 85.0% | SPECIFICITY 66.7% | |

**[Table 3]**

| sLOX-1≥334 pg/mL | | | |
|---|---|---|---|
| | MODERATE· SEVERE | MILD | PREDICTIVE VALUE |
| POSITIVE | 17 | 3 | 85.0% |
| NEGATIVE | 3 | 3 | 50.0% |
| | SENSITIVITY 85.0% | SPECIFICITY 50.0% | |

All the cut-off values resulted in positive predictive values as high as 85% or more. Each sensitivity was also 80% or more, suggesting the false negative rate of 20% or less. The above results revealed that setting the cut-off value to 330 pg/mL enables to determine that the severity is moderate nHIE or more if the measurement value is the cut-off value or more.

### (Prediction of good prognosis and determination results)

Tables 4 to 6 and Figure 2 show the results.

**[Table 4]**

| sLOX-1<1620 pg/mL | | | |
|---|---|---|---|
| | GOOD PROGNOSIS | POOR PROGNOSIS | PREDICTIVE VALUE |
| POSITIVE | 13 | 2 | 86.7% |
| NEGATIVE | 2 | 3 | 60.0% |
| | SENSITIVITY 86.7% | SPECIFICITY 60.0% | |

**[Table 5]**

| sLOX-1<1610 pg/mL | GOOD PROGNOSIS | POOR PROGNOSIS | PREDICTIVE VALUE |
|---|---|---|---|
| POSITIVE | 12 | 2 | 85.7% |
| NEGATIVE | 3 | 3 | 50.0% |
| | SENSITIVITY 80.0% | SPECIFICITY 60.0% | |

**[Table 6]**

| sLOX-1<1500 pg/mL | | | |
|---|---|---|---|
| | GOOD PROGNOSIS | POOR PROGNOSIS | PREDICTIVE VALUE |
| POSITIVE | 11 | 2 | 84.6% |
| NEGATIVE | 4 | 3 | 42.9% |
| | SENSITIVITY 73.3% | SPECIFICITY 60.0% | |

All the cut-off values resulted in positive predictive values as high as 84% or more. In other words, if the amount of the nHIE marker comprised in blood collected from a subject within a predetermined time after birth is less than the set cut-off value, it can be predicted that 84% or more of the case will have good prognosis if the subjects were treated by therapeutic hypothermia. Furthermore, the specificity (the percentage of the cases which is determined to be negative, that is, predicted as having poor prognosis, by the method of the present invention from among five subjects with poor prognosis) was 60% for all cut-off values. The above results revealed that setting the cut-off value to 1650 pg/mL enables to determine that the prognosis of a subject treated by therapeutic hypothermia will be good, if the measurement value is less than the cut-off value.

## Claims

1. A method for determining the severity of neonatal hypoxic-ischemic encephalopathy in a patient thereof, comprising:
a step of measuring the mass per unit volume of a neonatal hypoxic-ischemic encephalopathy marker consisting of a soluble LOX-1 protein or a polypeptide consisting of successive 6 to 182 amino acids comprised in the sLOX-1 protein shown in SEQ ID NO: 2 comprised in a blood sample that has been collected from a subject within 6 hours after birth, so as to obtain a measurement value; and
a step of determining the severity of neonatal hypoxic-ischemic encephalopathy in the subject based on the measurement value.

2. The method according to claim 1, wherein the severity of neonatal hypoxic-ischemic encephalopathy in the subject is determined to be moderate or more, when the measurement value is 330 pg/mL or more.

3. A method for determining the necessity of the application of therapeutic hypothermia to a neonatal hypoxic-ischemic encephalopathy patient, comprising:
a step of measuring the mass per unit volume of a neonatal hypoxic-ischemic encephalopathy marker consisting of a soluble LOX-1 protein or a polypeptide consisting of successive 6 to 182 amino acids comprised in the sLOX-1 protein shown in SEQ ID NO: 2 comprised in a blood sample that has been collected from a subject within 6 hours after birth, so as to obtain a measurement value; and
a step of determining the necessity of the application of therapeutic hypothermia to the subject based on the measurement value.

4. The method according to claim 3, wherein the application of therapeutic hypothermia to the subject is determined to be necessary, when the measurement value is 330 pg/mL or more.

5. A method for predicting the prognosis after treatment by therapeutic hypothermia, in a neonatal hypoxic-ischemic encephalopathy patient, comprising:
a step of measuring the mass per unit volume of a neonatal hypoxic-ischemic encephalopathy marker consisting of a soluble LOX-1 protein or a polypeptide consisting of successive 6 to 182 amino acids comprised in the sLOX-1 protein shown in SEQ ID NO: 2 comprised in a blood sample that has been collected from a subject within 6 hours after birth, so as to obtain a measurement value; and
a step of predicting the prognosis of the subject after treatment by therapeutic hypothermia, based on the measurement value.

6. The method according to claim 5, wherein the prognosis of the subject after treatment by therapeutic hypothermia is predicted to be good, when the measurement value is less than 1650 pg/mL.

## Patentansprüche

1. Verfahren zum Bestimmen des Schweregrades einer neonatalen hypoxischischämischen Enzephalopathie bei einem Patienten davon, umfassend:
einen Schritt des Messens der Masse pro Volumeneinheit eines Markers für neonatale hypoxisch-ischämische Enzephalopathie, bestehend aus einem löslichen LOX-1-Protein oder einem Polypeptid, bestehend aus aufeinanderfolgenden 6 bis 182 Aminosäuren, die in dem in SEQ ID NO: 2 gezeigten sLOX-1-Protein enthalten sind, das in einer Blutprobe enthalten ist, die innerhalb von 6 Stunden nach der Geburt von einem Subjekt entnommen wurde, um einen Messwert zu erhalten; und
einen Schritt des Bestimmens des Schweregrades der neonatalen hypoxischischämischen Enzephalopathie in dem Subjekt auf Grundlage des Messwerts.

2. Verfahren nach Anspruch 1, wobei der Schweregrad der neonatalen hypoxischischämischen Enzephalopathie in dem Subjekt als moderat oder mehr bestimmt wird, wenn der Messwert 330 pg/ml oder mehr beträgt.

3. Verfahren zum Bestimmen der Notwendigkeit der Anwendung von therapeutischer Hypothermie bei einem Patienten mit neonataler hypoxisch-ischämischer Enzephalopathie, umfassend:
einen Schritt des Messens der Masse pro Volumeneinheit eines Markers für neonatale hypoxisch-ischämische Enzephalopathie, bestehend aus einem löslichen LOX-1-Protein oder einem Polypeptid, bestehend aus aufeinanderfolgenden 6 bis 182 Aminosäuren, die in dem in SEQ ID NO: 2 gezeigten sLOX-1-Protein enthalten sind, das in einer Blutprobe enthalten ist, die innerhalb von 6 Stunden nach der Geburt von einem Subjekt entnommen wurde, um einen Messwert zu erhalten; und
einen Schritt des Bestimmens der Notwendigkeit der Anwendung von therapeutischer Hypothermie an dem Subjekt auf Grundlage des Messwerts.

4. Verfahren nach Anspruch 3, wobei die Anwendung von therapeutischer Hypothermie bei dem Subjekt als notwendig bestimmt wird, wenn der Messwert 330 pg/ml oder mehr beträgt.

5. Verfahren zum Vorhersagen der Prognose nach der Behandlung durch therapeutische Hypothermie bei einem Patienten mit neonataler hypoxisch-ischämischer Enzephalopathie, umfassend:
einen Schritt des Messens der Masse pro Volumeneinheit eines Markers für neonatale hypoxisch-ischämische Enzephalopathie, bestehend aus einem löslichen LOX-1-Protein oder einem Polypeptid, bestehend aus aufeinanderfolgenden 6 bis 182 Aminosäuren, die in dem in SEQ ID NO: 2 gezeigten sLOX-1-Protein enthalten sind, das in einer Blutprobe enthalten ist, die innerhalb von 6 Stunden nach der Geburt von einem Subjekt entnommen wurde, um einen Messwert zu erhalten; und
einen Schritt des Vorhersagens der Prognose des Subjekts nach der Behandlung durch therapeutische Hypothermie auf Grundlage des Messwerts.

6. Verfahren nach Anspruch 5, wobei die Prognose des Subjekts nach der Behandlung durch therapeutische Hypothermie als gut vorhergesagt wird, wenn der Messwert weniger als 1650 pg/ml beträgt.

## Revendications

1. Procédé de détermination de la sévérité d'une encéphalopathie hypoxique-ischémique néonatale chez un patient, comprenant :
une étape de mesure de la masse par unité de volume d'un marqueur d'encéphalopathie hypoxique-ischémique néonatale constitué d'une protéine LOX-1 soluble ou d'un polypeptide constitué de 6 à 182 acides aminés successifs compris dans la protéine sLOX-1 représentée dans la SEQ ID NO : 2 compris dans un échantillon de sang qui a été prélevé sur un sujet dans les 6 heures suivant la naissance, afin d'obtenir une valeur de mesure ; et
une étape de détermination de la gravité de l'encéphalopathie hypoxique-ischémique néonatale chez le sujet sur la base de la valeur de mesure.

2. Procédé selon la revendication 1, dans lequel la sévérité de l'encéphalopathie hypoxique-ischémique néonatale chez le sujet est déterminée comme étant modérée ou plus, lorsque la valeur de mesure est de 330 pg/mL ou plus.

3. Procédé pour déterminer la nécessité de l'application d'une hypothermie thérapeutique à un patient atteint d'encéphalopathie hypoxique-ischémique néonatale, comprenant :
une étape de mesure de la masse par unité de volume d'un marqueur d'encéphalopathie hypoxique-ischémique néonatale constitué d'une protéine LOX-1 soluble ou d'un polypeptide constitué de 6 à 182 acides aminés successifs compris dans la protéine sLOX-1 représentée dans SEQ ID NO : 2 compris dans un échantillon de sang qui a été prélevé sur un sujet dans les 6 heures suivant la naissance, de manière à obtenir une valeur de mesure ; et
une étape de détermination de la nécessité de l'application d'une hypothermie thérapeutique au sujet sur la base de la valeur de mesure.

4. Procédé selon la revendication 3, dans lequel l'application d'une hypothermie thérapeutique au sujet est déterminée comme étant nécessaire, lorsque la valeur de mesure est de 330 pg/mL ou plus.

5. Procédé de prédiction du pronostic après traitement par hypothermie thérapeutique, chez un patient atteint d'encéphalopathie hypoxique-ischémique néonatale, comprenant :
une étape de mesure de la masse par unité de volume d'un marqueur d'encéphalopathie hypoxique-ischémique néonatale constitué d'une protéine LOX-1 soluble ou d'un polypeptide constitué de 6 à 182 acides aminés successifs compris dans la protéine sLOX-1 représentée dans SEQ ID NO : 2 compris dans un échantillon de sang qui a été prélevé sur un sujet dans les 6 heures suivant la naissance, de manière à obtenir une valeur de mesure ; et
une étape de prédiction du pronostic du sujet après traitement par hypothermie thérapeutique, sur la base de la valeur de mesure.

6. Procédé selon la revendication 5, dans lequel le pronostic du sujet après traitement par hypothermie thérapeutique est prédit comme étant bon, lorsque la valeur de mesure est inférieure à 1650 pg/mL.
